# EUROPÄISCHE PATENTANMELDUNG

(11) **EP 4 494 756 A1**
(43) Veröffentlichungstag der Anmeldung: **22.01.2025**
(21) Anmeldenummer: 23186403.4
(22) Anmeldetag: 19.07.2023
(51) Int. Cl.: B01F 31/24, B01F 35/222, G01N 35/00

(54) **VORRICHTUNG UND LABORAUTOMAT ZUR INKUBATION VON MEHREREN PATIENTENPROBEN UNTER VERWENDUNG MEHRERER INKUBATIONSGEFÄSSE**

(71) Anmelder: EUROIMMUN Medizinische Labordiagnostika AG, 23560 Lübeck (DE)
(72) Erfinder: PETTER, Kolja, 23560 Lübeck (DE); MALZKUHN, Alexander, 23560 Lübeck (DE); REHM, Björn, 23560 Lübeck (DE)

(57) **Zusammenfassung**

Vorgeschlagen wird eine Vorrichtung zur Inkubation von mehreren Patientenproben unter Verwendung mehrerer Inkubationsgefäße, aufweisend eine erste Einheit mit jeweiligen Aufnahmen für jeweilige Inkubationsgefäße, sowie eine zweite Einheit mit jeweiligen Aufnahmen für jeweilige Inkubationsgefäße, wobei beide der jeweiligen Einheiten entlang einer gemeinsamen Linearführungsachse jeweils in ihrer jeweiligen Ortsposition bewegbar gelagert sind, ferner aufweisend jeweilige Sensoren zur Bereitstellung jeweiliger Sensorsignale, welche jeweils eine der jeweiliger Ortspositionen indizieren, ferner jeweilige Magnetantriebseinheiten für eine jeweilige Änderung einer jeweiligen Ortsposition, sowie ferner eine Steuereinheit, welche ausgebildet ist, auf Basis der jeweiligen Sensorsignale die jeweiligen Magnetantriebseinheiten zur Herbeiführung der jeweiligen Änderungen der jeweiligen Ortspositionen anzusteuern.

## Beschreibung

Die Erfindung betrifft eine Vorrichtung sowie einen Laborautomaten zur Inkubation von mehreren Patientenproben unter Verwendung mehrerer Inkubationsgefäße.

Für eine Vielzahl von technischen Verfahren auf den Gebieten der Biotechnologie, Pharmazie, Diagnostik sowie Medizin stellt sich die Aufgabe, mehrere bzw. jeweilige Patientenproben in jeweiligen Inkubationsgefäßen zu inkubieren. Eine Inkubation wird hierbei als ein Inkontaktbringen einer Patientenprobe mit einer oder mehrerer Flüssigkeiten eines sogenannten Assays bzw. Diagnostik-Assays verstanden. Hierfür muss die Patientenprobe mit einer oder mehrerer solcher Flüssigkeiten eines Assays nicht nur kurzzeitig in Kontakt gebracht werden, sondern das entsprechende Inkubationsgefäß muss für eine gewisse Zeitdauer die Patientenprobe sowie weitere Flüssigkeiten aufnehmen. Hierbei wird für eine Optimierung einer Vermischung der Patientenproben und solcher Flüssigkeiten das Inkubationsgefäß üblicherweise rhythmisch bewegt bzw. geschüttelt. Ferner kann auch vorzugsweise das Inkubationsgefäß hierbei einer vorbestimmten Temperatur ausgesetzt werden, welche dann innerhalb des Inkubationsgefäßes herrscht.

Für derartige Zwecke ist es aus logistischen Gründen und auch wirtschaftlichen Gründen erstrebenswert, einen Automatisierungsgrad solcher Inkubationsvorgänge zu erhöhen.

Bekannt sind Vorrichtungen, bei welchen ein einzelnes Inkubationsgefäß in eine Halterung eingebracht wird und wobei dann durch eine automatisierte Bewegung der Halterung eben auch das Inkubationsgefäß rhythmisch bewegt bzw. geschüttelt wird.

Aufgabe der vorliegenden Erfindung ist es, einen Automatisierungsgrad weiter zu erhöhen, so dass eine Inkubation von mehreren Patientenproben in entsprechenden bzw. jeweiligen Inkubationsgefäßen zumindest teilweise gleichzeitig stattfinden kann.

Die erfindungsgemäße Aufgabe wird gelöst durch eine Vorrichtung nach dem Anspruch 1 sowie einen Laborautomaten nach dem Anspruch 11.

Die erfindungsgemäße Vorrichtung zur Inkubation von mehreren Patientenproben weist eine erste Einheit mit jeweiligen, also mehreren, Aufnahmen für jeweilige Inkubationsgefäße auf sowie eine zweite Einheit mit jeweiligen, also mehreren, Aufnahmen für jeweilige Inkubationsgefäße.

Beide Einheiten sind entlang einer gemeinsamen Linearführungsachse jeweils in ihrer jeweiligen Ortsposition bewegbar gelagert.

Die Vorrichtung weist ferner jeweilige Sensoren zur Bereitstellung jeweiliger Sensorsignale auf, wobei die jeweiligen Sensorsignale jeweils eine der jeweiligen Ortspositionen einer jeweiligen Einheit indizieren.

Ferner weist die Vorrichtung jeweilige Magnetantriebe bzw. Magnetantriebseinheiten auf, welche jeweils ausgebildet sind, eine jeweilige Änderung einer jeweiligen Ortsposition einer jeweiligen Einheit zu bewirken bzw. herbeizuführen.

Schließlich weist die Vorrichtung ferner eine Steuereinheit auf, welche ausgebildet ist, auf Basis der jeweiligen Sensorsignale die jeweiligen Magnetantriebseinheiten zur Herbeiführung bzw. Bewirkung der jeweiligen Änderungen der jeweiligen Ortspositionen anzusteuern.

Die erfindungsgemäße Vorrichtung sieht es vor, dass zwei Einheiten jeweils mehrere Inkubationsgefäßaufnahmen aufweisen, so dass eine Mehrzahl an Inkubationsgefäßen zeitgleich einer rhythmischen Bewegung bzw. einem Schüttelvorgang ausgesetzt werden können. Dadurch, dass beide Einheiten entlang der gemeinsamen Linearführungsachse bewegbar gelagert sind, können diese beiden Einheiten sich nur entlang dieser Achse translatorisch bewegen, insbesondere nur entlang dieser Achse in Schwingung versetzt werden. Insbesondere sind die beiden Einheiten also in der Weise gelagert und geführt, dass sie nur einen gemeinsamen translatorischen Freiheitsgrad entlang der gemeinsamen Linearführungsachse besitzen. Hierdurch ist es prinzipiell möglich, dass die Massebewegungen der beiden Einheiten mit den darin befindlichen Inkubationsgefäßen nicht ungeordnet oder chaotisch stattfinden, sondern eben nur entlang der gemeinsamen Linearführungsachse, so dass prinzipiell diese beiden Massebewegungen sich gegenseitig kompensieren können, insbesondere wenn die Vorrichtung in einen gesamten Laborautomaten eingebaut wird.

Dadurch, dass die Steuereinheit auf Basis der jeweiligen Sensorsignale der jeweiligen Sensoren Kenntnis über die jeweiligen Ortspositionen der jeweiligen Einheiten besitzt, kann die Steuereinheit also über die jeweiligen Magnetantriebseinheiten in vorteilhafter Weise eine jeweilige Änderung einer jeweiligen Ortsposition an der jeweiligen Einheit bewirken. Würde nur eine einzelne Einheit zur Inkubation verwendet werden, so würde eine rhythmische Bewegung dieser einzelnen Einheit sich auf den gesamten Laborautomaten auswirken und somit auch andere Einheiten in dem Laborautomaten möglicherweise in Schwingung versetzen. Durch das Vorsehen zweier separater Einheiten, welche in Richtung der gemeinsamen Linearführungsachse bewegbar gelagert sind, kann eine solche Massebewegung einer einzelnen Einheit durch eine Massebewegung der zweiten Einheit kompensiert werden. Insbesondere steuert die Steuereinheit die Magnetantriebseinheiten also in solcher Weise an, dass eine Massebewegung der ersten Einheit durch eine Massebewegung der zweiten Einheit kompensiert wird. Hierdurch wird die gesamte Massebewegung der Gesamtmasse der Einheiten minimiert und somit eine mechanische Auswirkung auf den gesamten Laborautomaten ebenfalls minimiert.

Vorteilhafte Ausführungsform der Erfindung sind Gegenstand der abhängigen Ansprüche und werden in der Beschreibung unter teilweiser Bezugnahme auf die Figuren näher erläutert.

Vorzugsweise sind die beiden Einheiten jeweils in der Weise entlang der gemeinsamen Linearführungsachse gelagert und geführt, dass sie nur einen gemeinsamen translatorischen Freiheitsgrad besitzen. Hierdurch wird sichergestellt, dass die Massebewegungen der Einheiten nur entlang der einen Linearführungsachse bzw. des einen translatorischen Freiheitsgrades erfolgen und somit die Steuereinheit nur ausgebildet sein muss, um die Massebewegungen der jeweiligen Einheiten nur entlang dieses translatorischen Freiheitsgrades bzw. entlang dieser gemeinsamen Linearführungsachse so zu kontrollieren, dass die Massebewegungen sich gegenseitig zu wenigstens einem gewissen Grad kompensieren.

Vorzugsweise ist eine jeweilige Einheit über eine jeweilige Federeinheit mit einer gemeinsamen Grundplatte mechanisch gekoppelt. Hierbei bildet eine jeweilige Einheit mit einer jeweiligen Federeinheit zusammen vorzugsweise ein jeweiliges Feder-Masse-Schwingsystem aus. Diese Ausgestaltung ist vorteilhaft, da somit die Steuereinheit über die jeweiligen Magnetantriebseinheiten die jeweiligen Einheiten jeweils in eine Schwingung einer bestimmten Frequenz bringen kann. Hierbei kann die Steuereinheit den Frequenzgang eines jeweiligen Feder-Masse-Schwingsystems ausnutzen und muss vorzugsweise nur in solcher Weise Antriebsenergie über die Magnetantriebseinheiten erzeugen, welche möglichen Energieverlusten einer mechanischer Reibung und/oder einer Abweichungen der Schwingfrequenzen der Feder-Masse-Schwingsysteme von einer Zielfrequenz kompensieren. Die Steuereinheit ist also vorzugsweise ausgebildet, über die jeweiligen Magnetantriebseinheiten insbesondere die jeweiligen Einheiten in jeweilige Schwingungen gleicher Frequenz zu bringen.

Die Steuereinheit ist ferner vorzugsweise ausgebildet, die Magnetantriebseinheiten so anzusteuern, dass die Einheiten jeweils in Schwingung mit einer jeweils im Wesentlichen gleichen Schwingamplitude in Richtung der Linearführungsachse bzw. des translatorischen Freiheitsgrades in Schwingung gehalten werden. Die Schwingamplituden können auch als maximale Schwingamplituden bezeichnet werden.

Insbesondere ist die Steuereinheit vorzugsweise ausgebildet, die Magnetantriebseinheiten mittels eines Regelungsprinzips auf Basis der Sensorsignale so anzusteuern, dass die sich ergebenden jeweiligen Schwingfrequenzen der jeweiligen Einheiten im Wesentlichen gleich sind und dass die jeweiligen Schwingfrequenzen einen Phasenversatz von im Wesentlichen 180 Grad zueinander aufweisen. Besonders bevorzugt sind die sich ergebenden jeweiligen Schwingfrequenzen der jeweiligen Einheiten gleich und weisen einen Phasenversatz von 180 Grad zueinander auf. Diese Ausgestaltung ist insbesondere vorteilhaft, da dann eine Kompensation einer Massebewegung der ersten Einheit gegenüber einer Massebewegung der zweiten Einheit besonders optimiert bzw. ermöglicht wird, so dass insbesondere eine gesamte Auswirkung von Schwingungskräften der beiden gemeinsamen Einheiten auf einen Laborautomaten besonders minimiert wird.

Insbesondere ist die Steuereinheit ausgebildet, für eine jeweilige Einheit auf Basis eines jeweiligen Sensorsignals eine jeweilige aktuelle Amplitude bzw. Schwingungsamplitude und eine jeweilige Schwingfrequenz zu ermitteln, sowie ferner insbesondere beide Einheiten auf eine gemeinsame Schwingamplitude und eine gemeinsame, gleiche Ziel-Schwingfrequenz zu regeln. Hierdurch wird eine Optimierung der Schwingungskompensation der beiden Einheiten besonders gut erreicht. Die gemeinsame Schwingamplitude kann auch als gemeinsame maximale Schwingamplitude bezeichnet werden oder als maximale Ziel-Schwingamplitude.

Insbesondere weisen beide Einheiten vorzugsweise im unbeladenen Zustand eine gleiche Masse auf. Insbesondere sind die beiden Federeinheiten so ausgestaltet, dass sie eine gleiche, gemeinsame Federkonstante aufweisen.

Vorzugsweise weisen beide Einheiten mit den jeweils dazugehörigen Federeinheite ein jeweiliges Feder-Masse-Schwingsystem eine jeweils gleiche Resonanzfrequenz im unbeladenen Zustand auf, wobei eine Ziel-Schwingfrequenz der Regelung der Steuereinheit unterhalb dieser Resonanzfrequenz liegt. Diese Ausgestaltung ist vorteilhaft, da durch eine Beladung einer oder der beiden Einheiten mit Inkubationsgefäßen, und insbesondere darin befindlichen Flüssigkeiten, eine jeweilige Gesamtmasse einer jeweiligen Einheit erhöht wird und sich somit eine Resonanzfrequenz von dem unbeladenen Zustand des Systems hin zu einer etwas niedrigeren Resonanzfrequenz reduzieren wird. Dadurch, dass die Ziel-Schwingfrequenz der Regelung der Steuereinheit unterhalb der Resonanzfrequenz eines der Feder-Masse-Schwingsysteme im unbeladenen Zustand liegt, wird es somit erreicht, dass im beladenen Zustand der Einheiten die Ziel-Schwingfrequenz nahe einer Resonanzfrequenz der Feder-Masse-Schwingsysteme im beladenen Zustand der Einheiten liegt. Insbesondere bilden die jeweiligen Einheiten mit ihren jeweiligen Federeinheiten jeweils gleiche Feder-Masse-Schwingsysteme aus.

Vorzugsweise ist die Steuereinheit ferner ausgebildet, eine PI-Regelung bezogen auf eine gemeinsame, gleiche Ziel-Schwingfrequenz vorzunehmen. Ferner ist die Steuereinheit vorzugsweise ausgebildet, eine PI-Regelung bezogen auf eine gemeinsame, gleiche Ziel-Schwingfrequenz und eine gemeinsame, gleiche maximale Schwingamplitude für beide Einheiten vorzunehmen, wobei insbesondere die jeweiligen Schwingfrequenzen der jeweiligen Einheiten einen Phasenversatz von 180 Grad zueinander aufweisen. Diese Ausgestaltung ist vorteilhaft, da PI-Regelungen besonders stabil sind.

Vorzugsweise weist eine jeweilige Einheit eine jeweilige Heizeinheit auf. Diese Ausgestaltung ist vorteilhaft, da somit anzustrebende Temperaturen innerhalb der Inkubationsgefäße erreicht werden können.

Vorgeschlagen wird ferner ein Laborautomat aufweisend eine erfindungsgemäße Vorrichtung zur Inkubation. Der Laborautomat weist ferner ein Magazin mit einer Mehrzahl von Inkubationsgefäßen auf. Ferner weist der Laborautomat ein Behältnis mit Patientenprobenflüssigkeit, ein Behältnis mit Pufferflüssigkeit, ein Behältnis mit Bead-Flüssigkeit, wiederum aufweisend mit Antigenen oder Antikörpern beschichtete Beads, sowie ein Behältnis mit einer Label-Flüssigkeit aufweisend mit einem Chemilumineszenz-Label markierte Antikörper oder Antigene auf. Der Laborautomat ist somit insbesondere ein Laborautomat zur Prozessierung einer Patientenprobe unter Verwendung eines Chemilumineszenz-Assays zum Nachweis eines Ziel-Antigens oder eines Ziel-Antikörpers in der Patientenprobe.

Ferner weist der Laborautomat eine Pipettiereinheit auf, welche ausgebildet ist, um wenigstens einen Teil der Patientenprobenflüssigkeit, wenigstens einen Teil der Pufferflüssigkeit, wenigstens einen Teil der Bead-Flüssigkeit sowie wenigstens einen Teil der Label-Flüssigkeit aufweisend die mit einem Chemilumineszenz-Label markierte Antikörper oder Antigene in die Inkubationsgefäße zu dispensieren und aus den Inkubationsgefäßen zu aspirieren.

Der Laborautomat weist ferner eine Greifeinheit auf, welche ausgebildet ist, Inkubationsgefäße aus dem Magazin zur Pipettiereinheit zu transportieren sowie von der Pipettiereinheit in jeweilige der Aufnahmen zu transportieren, aus den jeweiligen Aufnahmen zu entnehmen und wieder hin zu der Pipettiereinheit zu transportieren. Der erfindungsgemäße Laborautomat ist vorteilhaft, da zum Zwecke einer Prozessierung eines Chemilumineszenz-Assays unter Verwendung von mit einem Chemilumineszenz-Label markierter Antikörper oder Antigene als ein Teil des Assays Patientenproben in jeweiligen Inkubationsgefäßen in den beiden Einheiten entsprechend des Chemilumineszenz-Assays inkubiert bzw. prozessiert werden können.

Vorzugsweise wird ferner ein Laborautomat vorgeschlagen, welcher eine der hierin offenbarten Ausführungsformen der Vorrichtung zur Inkubation aufweist.

Der Laborautomat weist vorzugsweise ferner ein Behältnis mit einem Enzym zur Umsetzung einer Chemilumineszenzreaktion auf sowie ferner eine Leseeinheit zur Erfassung eines optischen Signals einer Chemilumineszenzreaktion. Diese Ausgestaltung ist vorteilhaft, da dann automatisiert ein optisches Signal einer Chemilumineszenzreaktion herbeigeführt und erfasst werden kann.

Vorzugsweise sind die Magnetantriebe der Vorrichtung so angeordnet, dass in der Bead-Flüssigkeit enthaltene Beads nicht wesentlich durch ein Magnetfeld der Magnetantriebe magnetisch beeinflusst werden. Dieses ist vorteilhaft, da Beads üblicherweise magnetisierbare Partikel in Form metallischer Beads sind, welche während einer Durchmischung im Zuge der Inkubation nicht durch die Magnetantriebe in eine Vorzugsrichtung bzw. einen bevorzugten Ort bewegt werden sollen.

Im Folgenden wird die Erfindung anhand spezieller Ausführungsformen ohne Beschränkung des allgemeinen Erfindungsgedankens anhand der Figuren näher erläutert. Dabei zeigen:
- Figur 1A: eine Aufsicht auf eine beispielhafte erfindungsgemäße Vorrichtung
- Figur 1B: eine beispielhafte Detailansicht einer Einheit der Vorrichtung gemäß einer bevorzugten Ausführungsform
- Figur 2: eine schematische Darstellung der Vorrichtung gemäß einer bevorzugten Ausführungsform
- Figur 3: weitere Details einer bevorzugten Ausführungsform der Vorrichtung
- Figur 4: eine Prinzipdarstellung einer bevorzugten Ausführungsform eines erfindungsgemäßen Laborautomaten
- Figur 5A: ein Zusammenspiel eines Sensors mit einem Magneten
- Figur 5B: ein Kennlinienfeld mit Signalen
- Figur 6A sowie 6B: Details einer Greifeinheit im Zusammenspiel mit einem Inkubationsgefäß gemäß einer bevorzugten Ausführungsform
- Figur 7A sowie 7B: Details von Aufnahmen für Inkubationsgefäße sowie ein Inkubationsgefäß gemäß einer bevorzugten Ausführungsform
- Figuren 8A bis 8d: Signale und Frequenzverläufe
- Figur 9A: Details einer bevorzugten Ausführungsform einer Teil-Steuereinheit
- Figur 9B: weitere Details einer bevorzugten Ausführungsform einer Steuereinheit
- Figur 10: Details einer beispielhaften Regelung einer weiteren Teil-Steuereinheit

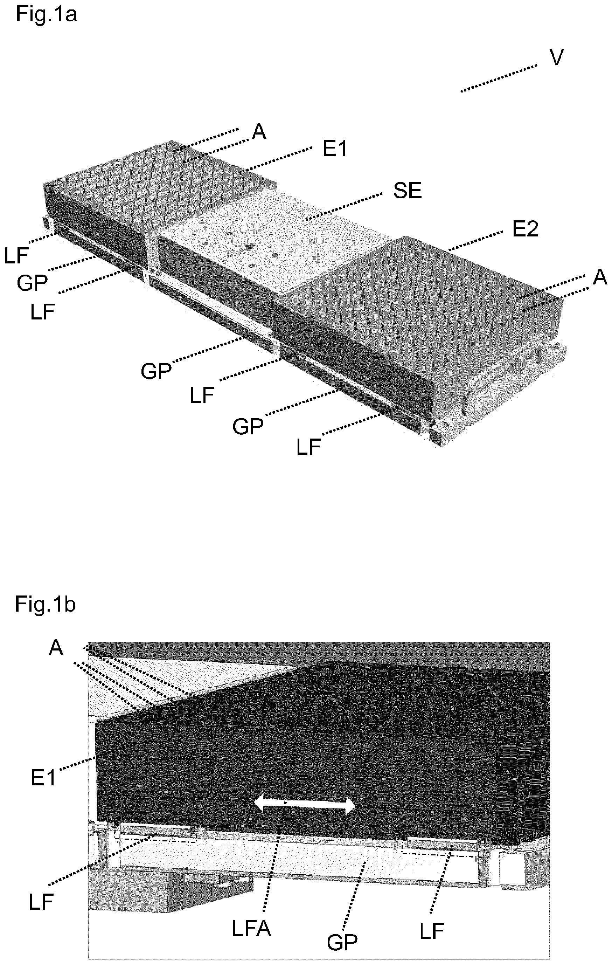

Die Figur 1A zeigt eine Vorrichtung V mit zwei Einheiten E1, E2, welche jeweilige Aufnahmen A für jeweilige Inkubationsgefäße aufweisen. Die Einheiten E1, E2 sind mittels Linearführungen LF entlang einer gemeinsamen Linearführungsachse LFA, siehe Figur 1B, bewegbar gelagert. Gezeigt ist ferner eine Steuereinheit SE. Die Einheiten E1, E2 sind auf einer gemeinsamen Grundplatte GP mittels der Linearführungselemente LF gelagert.

Die Figur 1B zeigt hierzu in einer Detailansicht die Einheit E1 sowie strichpunktiert umrandete Linearführungselemente LF und die Grundplatte GP.

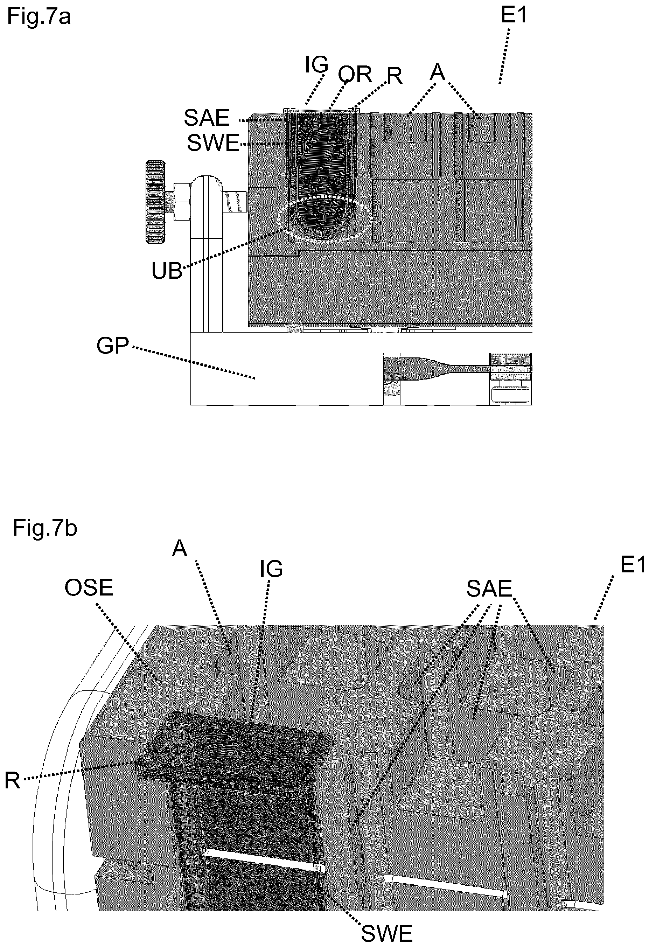

Die Aufnahmen A sind vorgesehen, um jeweilige Inkubationsgefäße aufzunehmen. Eine Detailansicht ist dargestellt in der Figur 7A in einer orthogonalen Schnittansicht, bei welcher ein Querschnitt durch die Aufnahmen A sichtbar wird sowie ein in eine Aufnahme A eingebrachtes Inkubationsgefäß IG. Eine entsprechende Perspektivansicht einer solchen Schnittdarstellung ist in der Figur 7B zu sehen, wobei auch hier das Inkubationsgefäß IG in eine Aufnahme A eingebracht wurde.

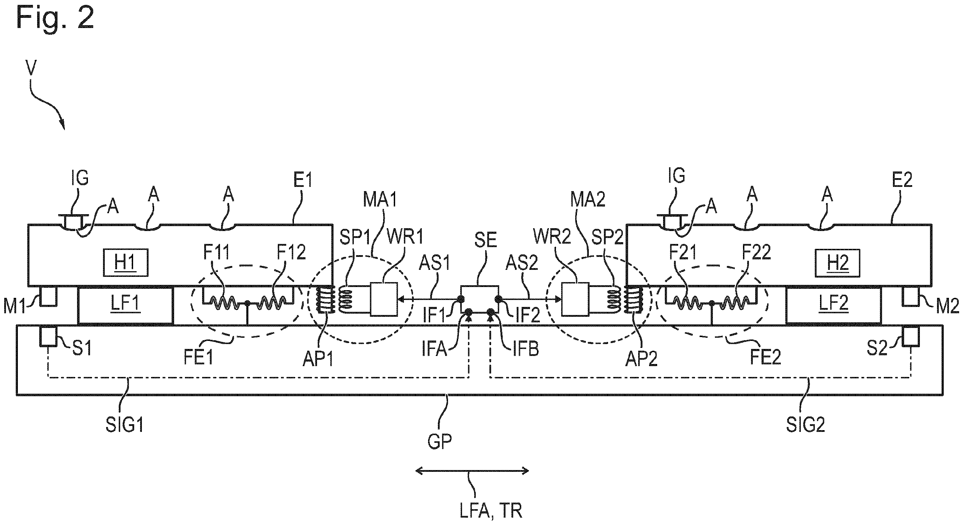

Die Figur 2 zeigt eine bevorzugte Ausführungsform der Vorrichtung V.

Die Einheiten E1, E2 sind mittels Linearführungseinheiten LF1, LF2 auf einer Grundplatte GP entlang einer gemeinsamen Linearführungsachse LFA gelagert. Die Einheiten E1, E2 weisen jeweilige Aufnahmen A für jeweilige Inkubationsgefäße IG auf. Vorzugsweise weisen die Einheiten E1, E2 jeweilige Heizeinheiten H1, H2 auf.

Die jeweiligen Einheiten E1, E2 sind mittels jeweiliger Federeinheiten FE1, FE2 mit der gemeinsamen Grundplatte GP mechanisch entkoppelt. Eine Federeinheit FE1 weist hierbei vorzugsweise zwei Federelemente F11, F12 auf. Eine Federeinheit FE2 weist hierbei vorzugsweise zwei Federelemente F21, F22 auf.

Mittels jeweiliger Sensoren S1, S2 können jeweilige Sensorsignale SIG1, SIG2 bereitgestellt werden, welche jeweils eine der jeweiligen Ortspositionen der jeweiligen Einheiten E1, E2 indizieren. Hierzu erfassen die jeweiligen Sensoren S1, S2 vorzugsweise jeweilige Magnetfelder, welche von jeweiligen Magneten M1, M2 erzeugt wurden.

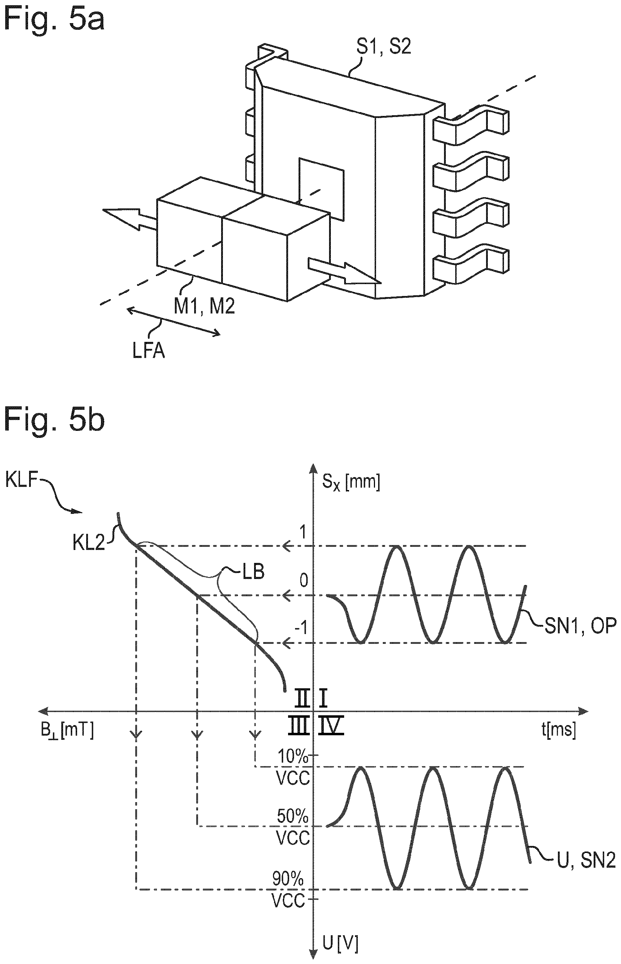

Ein Prinzip ist hierfür in der Figur 5A dargestellt, wobei ein Sensor S1, S2 insbesondere als ein Hall-Sensor ausgebildet ist, so dass eine translatorische Bewegung entlang der Linearführungsachse LFA eines Magneten M1, M2 erfasst werden kann. Ein Magnet M1, M2 ist insbesondere ein Diametralmagnet. Die Magneten M1, M2 sind gegenüber den jeweiligen Sensoren S1, S2 vorzugsweise in der Ruhelage mittig ausgerichtet bzw. positioniert.

Die Figur 5B zeigt ein Kennlinienfeld KLF, wobei in einem oberen rechten Quadranten I eine Ortsposition OP in Millimetern angegeben ist, welche bei einer mechanischen Schwingung einer entsprechenden Sinuskurve SN1 folgt. In einem oberen linken Quadranten II ist das sich ergebende Magnetfeld des Gebermagneten M1, M2 dargestellt. Bei Ausnutzung eines Linearbereiches LB der entsprechenden Kennlinie KL2 des Magnetfeldes kann dann eine lineare Umsetzung der Ortsposition OP ausgenutzt werden, so dass sich in dem linken unteren Quadranten III dann eine entsprechende Hall-Spannung ergibt, welche gemäß dem unteren rechten Quadranten IV in ein entsprechendes elektrisches Signal bzw. eine entsprechende Spannung U als eine Sinuskurve SN2 umgesetzt werden kann.

Ein derartiges Spannungssignal U kann dann als ein entsprechendes Signal SIG1, SIG2 gemäß der Figur 2 an die Steuereinheit SE bereitgestellt werden.

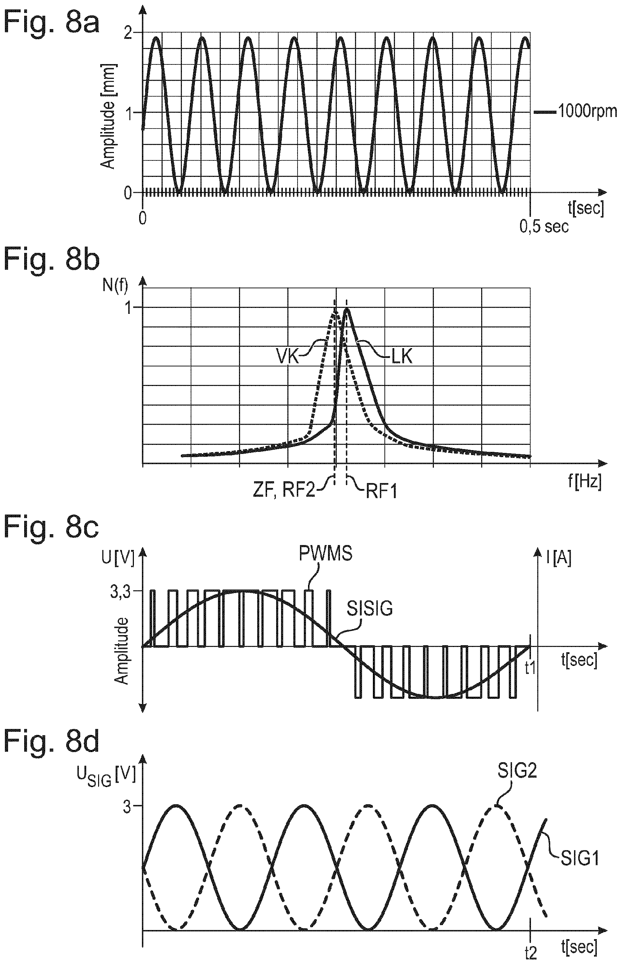

Die Steuereinheit SE kann mittels jeweiliger Ansteuerungssignale AS1, AS2 jeweilige Magnetantriebseinheiten MA1, MA2 ansteuern. Ein jeweiliges Ansteuerungssignal AS1, AS2 ist vorzugsweise jeweils ein pulsweitenmoduliertes Signal PWMS, wie in Figur 8C dargestellt. Hierbei ist auf der x-Achse die Zeit in Sekunden aufgetragen, wobei der Zeitpunkt t1 vorzugsweise einem Wert von t1=(1/16,67) Sekunden entspricht, da die anzusteuernde Schwingfrequenz vorzugsweise 16,67 Hz beträgt. Auf der y-Achse ist die Spannung U des pulsweitenmoduliertes Signals PWMS aufgetragen. Ein solches Signal PWMS liegt an einem Ausgang bzw. einer Schnittstelle der Steuereinheit an, welche bevorzugt einen Microcontroller aufweist. Der maximale Signalpegel eines solchen pulsweitenmodulierten Signals PWMS beträgt vorzugsweise 3,3 Volt.

Wird ein solches pulsweitenmoduliertes Signal PWMS als ein Ansteuerungssignal AS1, AS2 auf einen Wechselrichter WR1, WR2 gegeben, welcher an einer jeweiligen Spule SP1, SP2 angeschlossen ist, so ergibt sich dann durch die Spule ein entsprechendes sinusförmiges Stromsignal SISIG, wie in Figur 8C dargestellt. Auf der y-Achse ist ebenso die Stromstärke I des Stromsignals SISIG in Ampere aufgetragen. Aus einem solchen Stromsignal SISIG ergibt sich dann ein entsprechendes Magnetfeldsignal in der Nähe der Spule. Das pulsweitenmodulierte Signal PWMS und das Stromsignal SISIG können insbesondere einen Phasenversatz zueinander aufweisen.

Ein solch entsprechendes Magnetfeld kann dann eben genutzt werden, so dass eine jeweilige Magnetantriebseinheit MA1, MA2 eine jeweilige magnetisierbare Anschlussplatte bzw. Rückflussplatte AP1, AP2 entlang der Linearführungsachse LFA bewegt. Vorzugsweise führt eine solche Magnetisierung einer Anschlussplatte AP1, AP2 durch die Magnetantriebseinheiten MA1, MA2 zu Änderungen der jeweiligen Ortspositionen der jeweiligen Einheiten E1, E2. Eine jeweilige Einheit E1, E2 weist also eine jeweilige Anschlussplatte bzw. Rückflussplatte AP1, AP2 auf, welche durch eine jeweilige Magnetantriebseinheit MA1, MA2 angezogen oder abgestoßen werden kann.

Durch ein jeweiliges Federpaket FE1, FE2 bildet eine jeweilige Einheit E1, E2 ein Feder-Masse-Schwingsystem aus, wobei insbesondere die jeweiligen Einheiten E1, E2 über die jeweiligen Federpakete FE1, FE2 mit einer gemeinsamen Grundplatte GP mechanisch gekoppelt sind. Es ist somit bei Kopplung der Einheiten E1, E2 über die Federeinheiten FE1, FE2 mit der gemeinsamen Grundplatte GP prinzipiell möglich, dass sich beide Einheiten E1, E2 entlang der Linearführungsachse LFA bzw. translatorischen Richtung TR so bewegen, dass ihre jeweiligen Bewegungen gegenläufig verlaufen bzw. um 180 Grad versetzt verlaufen und somit sich diese jeweiligen Bewegungen gegenseitig kompensieren. Die Federeinheiten FE1, FE2 werden vorzugsweise durch Druckfedern F11, F12, F21, F22 ausgebildet.

Eine Einheit E1, E2 weist vorzugsweise eine Masse von 2,5kg auf. Eine Federeinheit FE1, FE2 weist vorzugsweise eine Federkonstante von ungefähr 11 N/mm² bis 12 N/mm² auf. Eine Einheit E1, E2 führt hierbei vorzugsweise eine sinusförmige Schwingung durch, welche in der Figur 8A dargestellt ist. Auf der Y-Achse ist eine Schwingungsamplitude bzw. Ortsposition in Millimetern aufgetragen und auf der x-Achse die Zeit in Sekunden.

Die Ausbildung als ein Feder-Masse-Schwingsystem mit Magnetantrieb ist energetisch effizienter als ein rein magnetisch angetriebenes System. Für ein rein magnetisch angetriebenes System ohne Federn würde für eine Änderung der Ortsposition zur Erzeugung einer Schwingung eine Ansteuerung der Magnetantriebe durch die Steuereinheit mehr Antriebsenergie erfordern. Ein Feder-Masse-Schwingsystem kann zunächst in Schwingung versetzt werden und es muss dann nur noch Energie aufgebracht werden, um die Schwingung aufrecht zu erhalten.

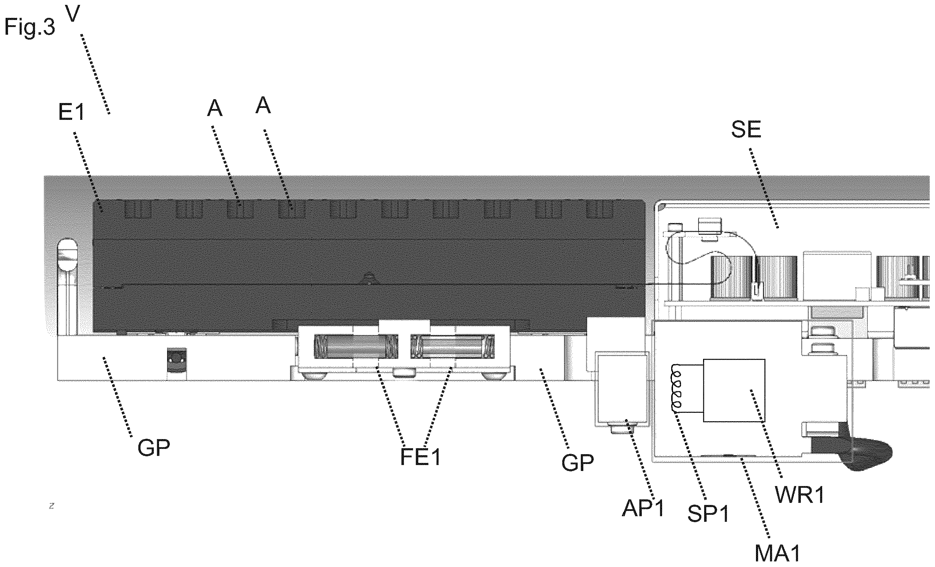

Da insbesondere ein Chemilumiszenz-Assay mit Bead-Flüssigkeit aufweisend magnetisierbare Beads in den Inkubationsgefäßen sich befindet, dürfen die Beads nicht magnetisch angezogen werden, so dass eine Herbeiführung der Schwingbewegung durch ein Feder-Masse-Schwingsystem besonders begünstigt wird, so dass eben nicht besonders hohe magnetische Felder bzw. Kräfte aufgebaut werden müssen, welche sich negativ auf eine Positionierung der magnetisierbaren Beads in den Inkubationsgefäßen auswirken könnten. Figur 3 zeigt hierzu noch einmal eine besondere Schnittansicht einer bevorzugten Ausführungsform einer Vorrichtung V mit einer Einheit E1 sowie entsprechenden Aufnahmen A, wobei über die Federeinheit FE1 die Einheit E1 mit der Grundplatte GP mechanisch gekoppelt ist. Die Steuereinheit SE steuert den Schwingmagneten aufweisend eine Spule SP1 und einen Wechselrichter WR1 an. Die Rückflussplatte AP1 kann somit zur Beeinflussung einer Schwingfrequenz der Einheit E1 entsprechend mittels des sich ergebenden Magnetfeldes der Spule SP1 beeinflusst werden. Insbesondere werden hierbei mechanische Reibungsverluste minimiert, um die Schwingfrequenz bzw. Ziel-Schwingfrequenz aufrecht zu erhalten.

Die Einheiten E1, E2 sind mittels der Heizeinheiten H1, H2 vorzugsweise auf 37 Grad Celsius beheizbar. Vorzugsweise weist eine Einheit E1, E2 jeweils 100 Aufnahmen A für Reaktionsgefäße bzw. Inkubationsgefäße IG auf. Für eine gute Durchmischung der Reagenzien des Assays werden die Inkubationsplatten entsprechend in Schwingung gebracht. Die Zielfrequenz einer solchen Schwingung beträgt vorzugsweise 16 Hz bis 17 Hz und die Schwingungsamplitude vorzugsweise 1 mm. Die in den Reagenzien bzw. der Bead-Flüssigkeit enthaltenen magnetischen Beads weisen eine höhere Dichte als eine Flüssigkeit auf, in welcher sich die Beads innerhalb der Inkubationsgefäße befinden. Daher wird durch die Frequenz und die Amplitude der Schwingung eine Sedimentierung der Beads verhindert, vorzugsweise gleichzeitig aber ein Herausgreifen eines Inkubationsgefäßes durch eine Greifeinheit im laufenden Betrieb ermöglicht.

Der vorgeschlagene Laborautomat LA ist also vorzugsweise ein sogenanntes Random Access Gerät, bei welchem eine Greifeinheit G im laufenden Betrieb bzw. bei fortlaufender Schwingung der Einheiten E1, E2 der Vorrichtung V Inkubationsgefäße IG im laufenden Betrieb in entsprechende Aufnahmen A einbringen kann und auch wieder herausgreifen.

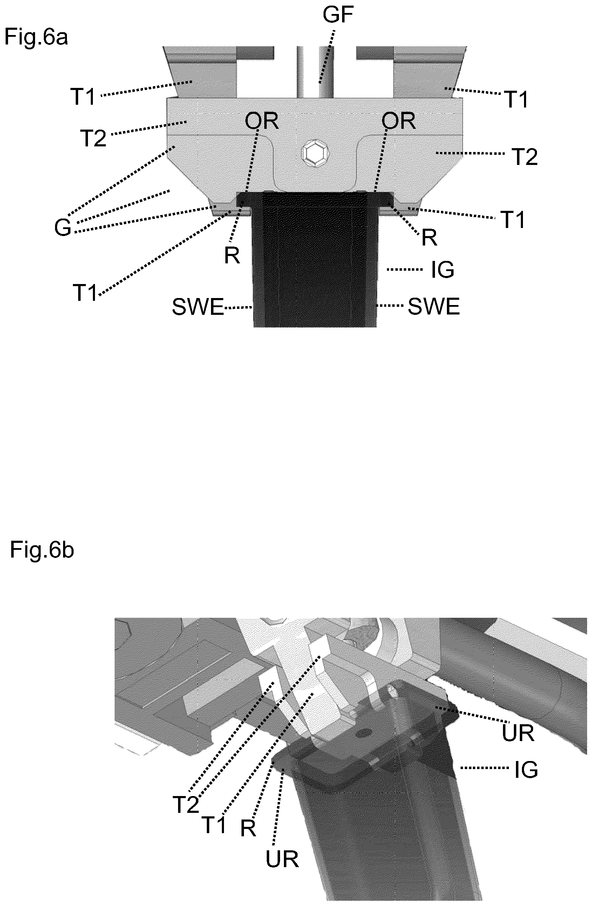

Dieses wird insbesondere dadurch erreicht, dass die Greifeinheit eine Kraft auf einen Rand R eines Inkubationsgefäßes IG, siehe Figuren 6A und 6B, von oben her durch eine Teileinheit T2 und von unten her durch eine Teileinheit T1 aufbringen kann, so dass in translatorischer Richtung lediglich Reibkräfte generiert werden, mittels welcher ein Inkubationsgefäß IG in einer Greifeinheit G gehalten wird. In anderen Richtungen als der translatorischen Richtung erfolgt insbesondere ein formschlüsssiges Greifen. Dadurch, dass in translatorischer Richtung ein reibschlüssiges Greifen erfolgt - und eben insbesondere bevorzugt kein formschlüssiges Greifen - kann das Inkubationsgefäß IG während des Griefvorgangs weiterhin minimale Bewegungen in einer Aufnahme A und in translatorischer Richtung ausführen, welche insbesondere durch eine Bewegung der Einheiten E1, E2 erzeugt werden, bis insbesondere ein Inkubationsgefäß IG so weit nach oben hin aus der Aufnahme A herausgenommen wurde, bis insbesondere genügend Abstand zwischen dem chronisch zusammenlaufenden unteren Bereich UB des Inkubationsgefäßes IG und den Seitenwänden SAE der Aufnahm Ae entstanden ist, siehe auch Figuren 7A und 7B.

Die Figur 8B zeigt einen Frequenzgang in Form einer Kennlinie LK für einen Fall, in welchem eine Einheit E1, E2 unbeladen ist. Auf der x-Achse ist eine Frequenz f in Herz aufgetragen und auf der Y-Achse ein normierter Frequenzgang N(f). Der Wert N(f)=1 entspricht einem Maximalwert des Frequenzgangs N(f) für den Fall, dass die Einheit E1, E2 unbeladen ist. Eine entsprechende Resonanzfrequenz RF1 ist ebenfalls eingezeichnet, welche vorzugsweise bei f=17 Hz liegt. Ferner ist ein Frequenzgang in Form einer Kennlinie VK für den Fall gezeigt, dass eine Einheit E1, E2 mit vollen Inkubationsgefäßen vollständig beladen ist. Eine entsprechende Resonanzfrequenz RF2 bzw. Zielfrequenz ZF ist ebenfalls eingezeichnet, welche vorzugsweise bei f=16,67 Hz liegt.

Für die Ansteuerung der Magnetantriebseinheiten MA1, MA2 durch die Steuereinheit SE ist eine Zielfrequenz ZF vorgesehen, welche niedriger als die Resonanzfrequenz RF1 ist. Eine entsprechende Kennlinie ist als die Kennlinie VK vorgesehen. Die Kennlinie VK zeigt beispielhaft einen Frequenzgang mit einer Resonanzfrequenz RF2 für den Fall, dass der Inkubator bzw. die Einheit E1, E2 vollbeladen ist mit Inkubationsgefäßen entsprechender Reagenzien.

Durch Auswahl einer Zielfrequenz ZF, so dass die Zielfrequenz ZF unterhalb der Resonanzfrequenz RF1 der Einheiten E1, E2 im unbeladenen Zustand liegt, wird in vorteilhafter Weise im Zuge der Ansteuerung bzw. Regelung der Fall abgebildet, dass sich weitere Massen bzw. Massenelemente in Form der Inkubationsgefäße in den Einheiten E1, E2 befinden können und somit die notwendige Energie zur Aufrechterhaltung einer entsprechenden Zielfrequenz ZF weiter minimiert wird.

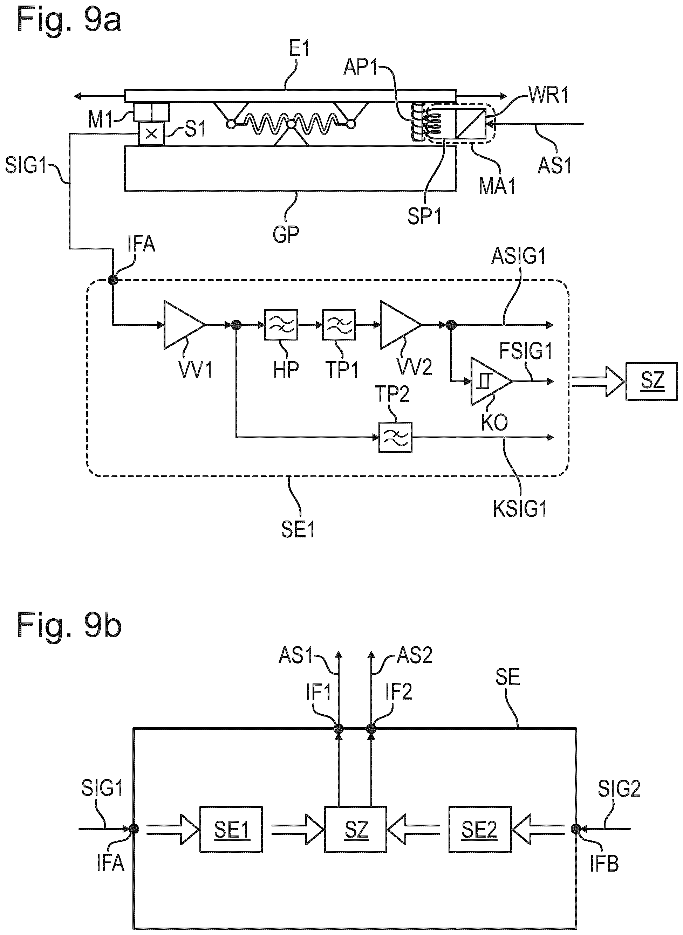

In der Figur 2 ist das Steuergerät SE dargestellt, welches auf Basis der Sensorsignale SIG1, SIG2 Ansteuersignale AS1, AS2 bestimmt, um die Magnetantriebseinheiten MA1, MA2 anzusteuern. Die Figur 9B zeigt hierbei eine bevorzugte Ausführungsform der Steuereinheit SE, welche Teil-Steuereinheiten SE1, SE2 sowie SZ aufweist.

Das Sensorsignal SIG1 wird an einer Schnittstelle IFA entgegengenommen. Ferner weist das Steuergerät SE eine Schnittstelle IFB zum Entgegennehmen des Sensorsignals SIG2 auf. Mittels einer Untereinheit SE1 wird das Sensorsignal SIG1 aufbereitet und verarbeitet. Anschließend werden die ermittelten Größen bzw. Signale werden an die zentrale Teil-Steuereinheit SZ bereitgestellt.

Gleiches findet in der Teil-Steuereinheit SE2 auf Basis des Signals SIG2 statt. Die zentrale Teil-Steuereinheit SZ ermittelt dann die Ansteuersignale AS1, AS2 und stellt diese an entsprechenden Schnittstellen IF1 bzw. IF2 bereit.

Die Ansteuersignale AS1, AS2 sind pulsweitenmodulierte Signale, wie als Signal PWMS in der Figur 8c dargestellt. Die Ansteuersignale AS1, AS2 sind bezüglich ihres Duty-Cycles synchronisiert und werden lediglich in ihrer Pulsweite moduliert.

Die Figur 9A zeigt hierzu Details einer Teil-Steuereinheit SE1. Die Einheit E1 ist über die Federeinheit FE1 an einer Grundplatte GP wie zuvor beschrieben mechanisch gekoppelt. Der Magnet M1 wird bezüglich seiner Position mittels des Sensors S1 überwacht bzw. detektiert. Der Sensor S1 gibt das Sensorsignal SIG1 als ein Spannungssignal aus. An der Schnittstelle IFA nimmt die Teil-Steuereinheit SE1 das Sensorsignal SIG1 entgegen. Die Teil-Steuereinheit SE1 leitet aus dem Sensorsignal SIG1 ein Amplitudenmesssignal ASIG1 ab und ferner vorzugsweise ein Frequenzmesssignal FSIG1. Besonders bevorzugt ermittelt die Teil-Steuereinheit SE1 auf Basis des Sensorsignals SIG1 ferner ein Kalibrationssignal KSIG1 für ein Bereitstellen eines Signals indizierend eine Nullposition im Zuge einer Teaching-Prozedur vor finaler Inbetriebnahme der Vorrichtung. Das Sensorsignal SIG1 wird durch einen Vorverstärker VV1 verstärkt. Es folgt dann vorzugsweise ein Hochpass HP gefolgt von einem Tiefpass TP1, so dass ein Bandpass ausgebildet wird. Das sich ergebende Signal wird dann durch einen weiteren Vorverstärker VV2 verstärkt, so dass sich das Amplitudenmesssignal ASIG1 ergibt. Aus dem Amplitudenmesssignal kann dann ferner mittels eines Komparators KO vorzugsweise das Frequenzmesssignal FSIG1 ermittelt werden. Das durch den Vorverstärker VV1 verstärkte Sensorsignal SIG1 kann ferner vorzugsweise mittels eines Tiefpassfilters TP2 gefiltert werden, um das Kalibrationssignal KSIG1 zu ermitteln. Die ermittelten Signale ASIG1, FSIG1, KSIG1 können dann an die zentrale Teil-Steuereinheit SZ SZ bereitgestellt werden. Vorzugsweise werden wenigstens die ermittelten Signale ASIG1, Amplitudensignal, und FSIG1, Frequenzsignal, an die zentrale Steuereinheit SZ bereitgestellt.

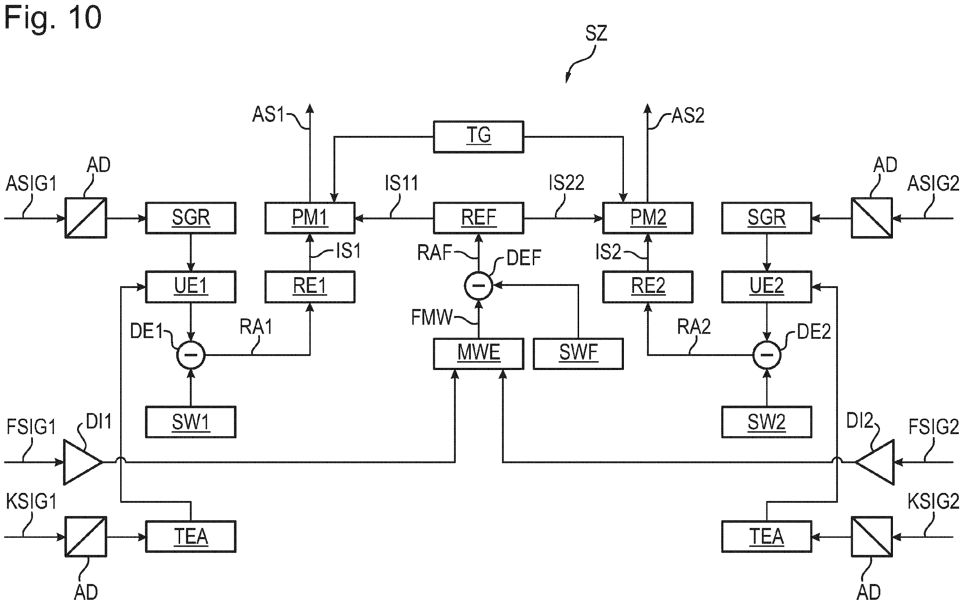

Die Teil-Steuereinheit SE2 aus der Figur 9B ermittelt auf Basis des zweiten Sensorsignals SIG2 entsprechende Signale in Form eines Amplitudenmesssignals, eines Frequenzmesssignals sowie vorzugsweise eines Kalibrationssignals und stellt auch diese an die zentrale Teil-Steuereinheit SZ bereit. Weitere Details sind der Figur 10 zu entnehmen, welche eine Ausführungsform der Teil-Steuereinheit SZ illustriert.

Ziel der Ermittlung der Ansteuersignale AS1, AS2 ist es, dass die sich ergebenden Ortspositionen bzw. Schwingungsamplituden der beiden bewegten Einheiten so verhalten, wie durch entsprechende Signale SIG1, SIG2 in der Figur 8D dargestellt. Auf der x-Achse ist die Zeit dargestellt, wobei vorzugsweise der Zeitpunkt t2 einem Wert von t1=(3/16,67) Sekunden entspricht, da die anzusteuernde Schwingfrequenz vorzugsweise 16,67 Hz beträgt. Auf der y-Achse ist die Sensorspannung USIG in Volt aufgetragen. Die sich ergebenden jeweiligen Schwingfrequenzen der jeweiligen Einheiten weisen dann ein Phasenversatz von 180 Grad zueinander auf. Hierfür ermittelt die Steuereinheit anhand der Teil-Steuereinheit SE1 also auf Basis des Sensorsignals SIG1 ein Amplitudenmesssignal bzw. eine Amplitude ASIG1 sowie eine Schwingfrequenz bzw. ein Frequenzmesssignal FSIG1. Ferner ermittelt die Teil-Steuereinheit SE2 auf Basis des Sensorsignals SIG2 ein Amplitudenmesssignal bzw. eine Amplitude ASIG2 sowie eine Schwingfrequenz bzw. ein Frequenzmesssignal FSIG2 sowie vorzugsweise ein Kalibrationssignal KSIG2. Die Steuereinheit ermittelt dann insbesondere mittels der Teil-Steuereinheit SZ die Ansteuersignale AS1, AS2 in der Art, dass beide Einheiten auf eine gemeinsame Schwingamplitude und eine gemeinsame, gleiche Ziel-Schwingfrequenz geregelt werden.

Die Schwingfrequenzen haben daneben insbesondere einen Phasenversatz von 180 Grad zueinander. Die durch die Steuereinheit SE durchgeführte Ansteuerung ist eine Regelung, insbesondere eine PI-Regelung bezogen auf eine gemeinsame, gleiche Ziel-Schwingfrequenz.

Das Amplitudenmesssignal ASIG1 wird nach einer Analogdigitalwandlung durch einen AD-Wandler dann mittels eines Spitzenwertgleichrichters SGR gleichgerichtet. Durch eine Umrechnungseinheit UE1 erfolgt dann eine Umrechnung des digitalen Signals in Millimeter. Eine Differenzbildungseinheit DE1 nimmt von einer Sollwerteinheit SW1 einen Sollwert von vorzugsweise 1 mm entgegen und bestimmt dann als Differenz die Regelabweichung RA1, welche einer Regeleinheit RE1 zugeführt wird. Die Regeleinheit RE1 führt eine PI-Regelung durch, welche vorzugsweise als Werte einen KP-Wert von 0,03 und als KI-Wert einen Wert von 0,6 verwendet. Eine entsprechende Bestimmung einer Regelabweichung RA2 auf Basis des Sensorsignals ASIG2 erfolgt mittels eines AD-Wandlers AD, eines Spitzenwertgleichrichters SGR und einer Umrechnungseinheit KE2, wobei auch hier der Sollwert 1 mm beträgt, welchen die Sollwerteinheit SW2 vorgibt, so dass die Differenzeinheit RA2 die Regelabweichung RA2 bestimmt. Es erfolgt dann mit gleichen KP- und KI-Werten der Einheit RE1 eine PI-Regelung in der Regeleinheit RE2.

Das Frequenzmesssignal FSIG1 wird über einen digitalen Input DI1 an einer Mittelwerteinheit MWE bereitgestellt. Entsprechendes passiert bzw. wird vorgenommen durch Zuführung des Frequenzmesssignals FSIG2 über einen digitalen Input DI2 und Bereitstellung an die Mittelwerteinheit MWE. Eine Sollwerteinheit SWF stellt einen Sollwert von vorzugsweise 16,67 Hz bereit, so dass dann eine Differenzeinheit DEF eine Differenz zwischen dem Frequenzmittelwert FMW und der Sollgröße als eine Regelabweichung RAF bestimmt. Eine Regeleinheit REF bestimmt auf Basis des Eingangssignals bzw. der Regelabweichung RAF mittels einer PI-Regelung und Parameterwerten von KP=0,009 und KI=0,09 eine PI-Regelung in der Weise, dass jeweilige interne Steuersignale IS11 bzw. IS22 an die jeweiligen Modulatoren PM1 bzw. PM2 bereitgestellt werden.

Ein Synchrontaktgeber TG steuert entsprechende Pulsweitenmodulatoren PM1 bzw. PM2 an. Der Pulsweitenmodulator PM1 wird hinsichtlich seiner Pulsbreite durch ein Signal IS1 der Amplitudenregelungseinheit RE1 und ein Signal IS11 der Frequenzregelungseinheit REF angesteuert und erzeugt dann das Ansteuersignal AS1. Der Pulsweitenmodulator PM2 wird hinsichtlich seiner Pulsbreite durch ein Signal IS2 der Amplitudenregelungseinheit RE2 und ein Signal IS22 der Frequenzregelungseinheit REF angesteuert und erzeugt dann das Ansteuersignal AS2.

Wie bereits zuvor ausgeführt, ist in einer Detailansicht der Figur 7A eine orthogonale Schnittansicht dargestellt, bei welcher ein Querschnitt durch die Aufnahmen A sichtbar wird sowie ein in eine Aufnahme A eingebrachtes Inkubationsgefäß IG. Eine entsprechende Perspektivansicht einer solchen Schnittdarstellung ist in der Figur 7B zu sehen, wobei auch hier das Inkubationsgefäß IG in einer Aufnahme A eingebracht wurde.

Das Inkubationsgefäß IG läuft vorzugsweise in einem unteren Bereich UB verjüngend zusammen und weist in seinem oberen Bereich einen umlaufenden Rand R auf, welcher über die Seitenwände SWE des Gefäßes übersteht.

Das Inkubationsgefäß IG kommt bei Einbringung in eine Aufnahme A mit seinem Rand R auf einer Oberseite OSE der Einheit E1 zum Aufliegen. Hierdurch wird das Inkubationsgefäß IG in der Aufnahme A zumindest teilweise gehalten. Ferner wird das Inkubationsgefäß IG vorzugsweise dadurch gehalten, dass es zumindest teilweise zu einem Formschluss zwischen der Aufnahme A und dem Inkubationsgefäß IG kommt.

Die Figur 6A zeigt ein Inkubationsgefäß IG mit seinem Rand R sowie den Seitenwänden SWE, wobei das Inkubationsgefäß IG von einer Greifeinheit G gehalten wird. Eine solche Darstellung ist in einer Schrägansicht auch noch in der Figur 6B dargestellt.

Ein erster Teil T1 eines Klemmmechanismus der Greifeinheit G fasst auf zwei Seiten unterhalb des Randes R bzw. auf der Unterseite UR des Randes das Inkubationsgefäß IG. Ferner greift eine zweite Teileinheit T2 eines Klemmmechanismus der Greifeinheit G das Inkubationsgefäß dadurch, dass diese zweite Teileinheit T2 von oben her durch eine Federeinheit GF auf die Oberseite OR des Randes des Inkubationsgefäßes IG gedrückt wird. Es kommen somit die Teileinheiten T1 und T2 der Greifeinheit in ein Zusammenspiel zum Erzeugen einer Reibkraft zum Halten des Inkubationsgefäßes. Hierdurch kann die Greifeinheit G das Inkubationsgefäß IG greifen.

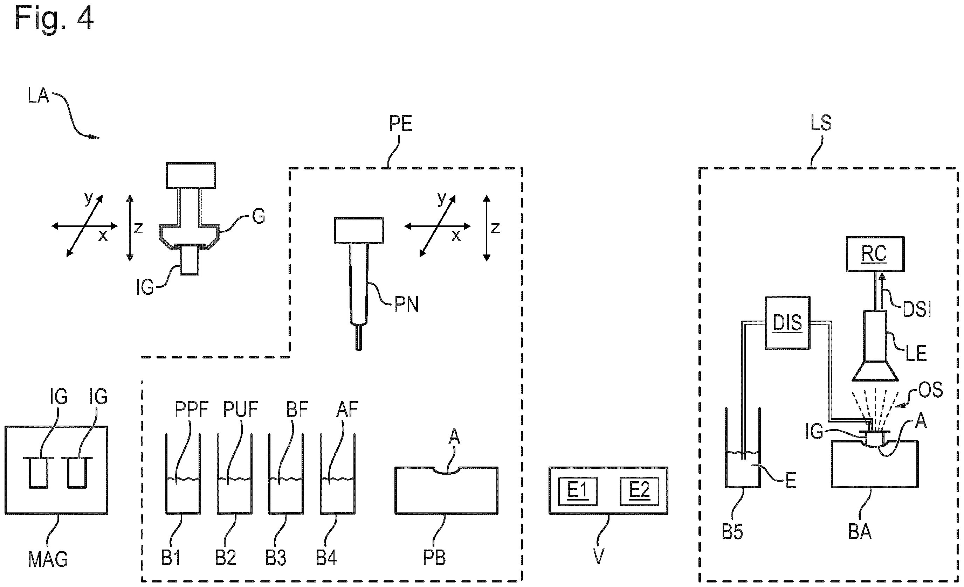

In der Figur 4 ist eine Prinzipskizze einer bevorzugten Ausführungsform eines erfindungsgemäßen Laborautomaten LA dargestellt. Der Laborautomat LA weist eine Greifeinheit G zum Greifen von Inkubationsgefäßen IG auf. Der Laborautomat LA weist ferner ein Magazin MAG mit mehreren Inkubationsgefäßen IG auf. Mittels der Greifeinheit G können Inkubationsgefäße IG von dem Magazin MAG hin zu einer Pipettiereinheit PE transportiert werden. In einer vorzugsweise vorgesehenen Pipettierbasis PB der Pipettiereinheit PE ist dann eine Aufnahme A für eine Aufnahme eines Inkubationsgefäßes IG vorgesehen. Die Pipettiereinheit PE kann also insbesondere in einer Aufnahme A ein Inkubationsgefäß IG aufnehmen.

Der Laborautomat LA weist ferner ein Behältnis B1 mit einer Patientenprobenflüssigkeit PPF auf. Der Laborautomat LA weist ferner ein Behältnis B2 mit einer Pufferflüssigkeit PUF auf. Der Laborautomat LA weist ferner ein Behältnis B3 mit einer Bead-Flüssigkeit BF auf. Der Laborautomat weist ferner ein Behältnis B4 mit einer Label-Flüssigkeit AF auf, welche mit einem Chemilumineszenzlabel markierte Antikörper oder mit einem Chemilumineszenzlabel markierte Antigene aufweist.

Der Laborautomat LA weist ferner eine erfindungsgemäße Vorrichtung V auf.

Die Greifeinheit G ist also ferner ausgebildet, Inkubationsgefäße IG von der Pipettiereinheit PE in jeweilige Aufnahmen der jeweiligen Einheiten E1, E2 der Vorrichtung V zu bringen und auch wieder zu entnehmen, insbesondere um auch Inkubationsgefäße wieder von der Vorrichtung V zu der Pipettiereinheit PE zu transportieren. Hierfür ist die Greifeinheit G vorzugsweise in X-Richtung, Y-Richtung sowie Z-Richtung verschiebbar bzw. bewegbar. Die Greifeinheit G kann Inkubationsgefäße IG automatisiert greifen und auch wieder freigeben bzw. ablegen.

Die Pipettiereinheit PE weist eine oder mehrere Pipettiernadeln PN auf, welche vorzugsweise in X-Richtung, Y-Richtung sowie Z-Richtung bewegbar bzw. verschiebbar sind. Mittels solcher Pipettiernadel PN können verschiedene der Flüssigkeiten PPF, PUF, WF, AF aus den Behältnissen B1, B2, B3, B4 in ein Inkubationsgefäß IG pipettiert werden oder hieraus aspiriert werden.

Zur Durchführung eines Chemilumineszenz-Assays weist der Laborautomat LA ferner vorzugsweise eine Lesestation LS auf. Somit weist der Laborautomat LA ferner vorzugsweise ein Behältnis B5 mit einer Flüssigkeit bzw.Triggerflüssigkeit E auf, welche wiederum ein Enzym zur Umsetzung in der Chemilumineszenzreaktion aufweist.

Vorzugsweise wird durch eine Dispensiereinheit DIS das Enzym E in ein Inkubationsgefäß IG eingebracht, welches sich vorzugsweise in einer Aufnahme A einer Basis BA befindet. Entstehende optische Strahlung bzw. ein entstehendes optisches Signal OS kann vorzugsweise mittels einer Leseeinheit LE erfasst werden. Vorzugsweise kann dann nach Erfassen des optischen Signals OS ein entsprechendes digitales Signal DSI durch eine Recheneinheit RC ausgewertet werden und vorzugsweise über eine Datenschnittstelle der Recheneinheit RC über ein Datennetzwerk versendet werden. Die Recheneinheit RC ist ein vorzugsweiser integraler Bestandteil des Laborautomaten LA. Die Lesestation LS weist also die Leseeinheit LE und die Recheneinheit RC auf.

Der Laborautomat ist insbesondere ein Laborautomat zur Prozessierung einer Patientenprobe unter Verwendung eines Chemilumineszenz-Assays zum Nachweis eines Ziel-Antigens oder eines Ziel-Antikörpers in der Patientenprobe.

Die Patientenprobe ist insbesondere eine flüssige Patientenprobe. Die Patientenprobenflüssigkeit kann eine homogene flüssige Phase aufweisen. Bevorzugt handelt es sich bei der Patientenprobenflüssigkeit um zur diagnostischen Untersuchung abgenommene und optional aufbereitete menschliche Proben, bevorzugt z.B. Blut, bevorzugt Blutserum, Urin, Liquor, Speichel oder Schweiß.

Für viele automatisierte Systeme werden zum Nachweis eines bestimmten Antigens bzw. Ziel-Antigens oder aber eines bestimmten Antikörpers bzw. Ziel-Antikörpers in einer Patientenprobe zum Erkenntnisgewinn hinsichtlich eines möglichen Gesundheitszustandes eines Patienten sogenannte Chemilumiszenz-Verfahren bzw. Chemilumiszenz-Assays eingesetzt. Hierbei werden insbesondere Beads als Träger für Reagenzien eingesetzt. Beispielsweise können die Beads Träger für immobilisierte Antigene sein, an die in menschlichen Proben nachzuweisende Antikörper bzw. Ziel-Antikörper binden. Die Beads werden üblicherweise in wässrigen Lösungen geliefert und bis zur Verwendung aufbewahrt, der sogenannten Bead-Flüssigkeit. Die Beads sind insbesondere magnetisierbare Partikel, insbesondere metallische, magnetisierbare Partikel. Werden derartige Beads mit einer flüssigen Patientenprobe inkubiert, so kommt es bei Anwesenheit der Ziel-Antikörper zur Bildung eines Antigen-Antikörper-Komplexes, der am Bead immobilisiert ist. Nach einem Waschschritt mit einer Spülflüssigkeit bzw. Pufferflüssigkeit kann dieser Komplex mit geeigneten Reagenzien, insbesondere Konjugat genannt, inkubiert werden. Dies sind insbesondere Label-Flüssigkeiten aufweisend mit einem Chemilumineszenzlabel, besonders bevorzugt in Form von Acridinium, markierter sekundäre Antikörper. Dieser sekundäre Antikörper ist vorzugsweise ein Anti-Human-Antikörper, vorzugsweise Anti-Human IgG Antikörper. Auf diesem Prinzip beruhen die im Handel erhältlichen sogenannten Chemilumineszenz-Laborautomaten, welche bevorzugt auch ein Random-Access-Analyzer sein können. Zur Erzeugung eines optischen Signals wird dann zum Schluss der Abarbeitung des Chemilumiszenz-Verfahrens eine Lösung bzw. Triggerlösung mit einem Enzym hinzugefügt, um eine Chemilumiszenz-Reaktion zwischen dem Enzym und dem Chemilumineszenzlabel herbeizuführen, so dass ein optisches Signal entsteht. Das entstehende optische Signal ist zur Antikörperkonzentration in der Patientenprobe und kann nach einer Erfassung vorzugsweise wird vom Laborautomaten automatisch in eine Konzentration umgerechnet werden. Die Label-Flüssigkeit wird insbesondere auch Konjugat-Flüssigkeit genannt.

Zum Nachweis eines Antigens statt eines Antikörpers werden antikörperbeschichtete Magnetpartikel, sogenannte Beads, mit der Patientenprobe und einem antigenspezifischen biotinylierten Antikörper inkubiert. Während der Inkubation wird das Antigen sowohl vom Magnetpartikel-gekoppelten als auch vom biotinylierten Antikörper gebunden. In einem weiteren Schritt erfolgt die Zugabe von Konjugat, das den biotinylierten Antikörper bindet. Anschließend wird der Reaktionsansatz mit der Triggerlösung versetzt, die eine Chemilumineszenz-Reaktion induzieren.

Immundiagnostische Tests bzw. Assays, die nach diesem Prinzip funktionieren, sind für zahlreiche Indikationen im Stand der Technik beschrieben, beispielsweise in der EP 2 199 303, DE 10 2009033281, WO 2010/009457 oder EP12183919.5. Prinzipien eines Chemilumineszenz-Verfahrens sind ebenfalls in der EP 3 160 646 B1 beschrieben.

Geeignete Reagenzien sind im Stand der Technik beschrieben, beispielsweise in Ireland, D., und Samuel, D. (1989), "Enhanced Chemiluminscence ELISA for the detection for antibody to hepatitis B virus surface antigen", J. Biolum. Chemilumin., 159-163 und in "Acridinium Esters as Highly Specific Activity Labels in Immunoassays," Clin. Chemistry 19:1474-1478 (1984) und in US4842997 A.

Ein besonderes Problem stellen inhomogene Bead-Flüssigkeiten dar, beispielsweise Suspensionen von Beads in wäßriger Lösung, deren Dichte höher ist als die von Wasser, so dass die Beads zu Boden sinken können. Im Zuge der Inkubation kann es in einem Inkubationsgefäß zu einer Sedimentierung der Beads kommen. Alternativ kann es dazu kommen, dass sich derartige Beads in flüssiger Phase leicht an Oberflächen ansammeln. Daher werden die Inkubationsgefäße während der Inkubation regelmäßig bewegt bzw. in Schwingung gebracht.

Die Bead-Flüssigkeit kann aber auch eine inhomogene Phase aufweisen und entweder zwei nicht oder nur begrenzt mischbare Flüssigkeiten oder einen festen Stoff in einer Flüssigkeit umfassen. In einer bevorzugten Ausführungsform handelt es sich bei der Flüssigkeit um Beads in wässriger Lösung. Solche Beads können mit daran immobilisierten biologischen Reagenzien versehen sein, z. B. als Antigen fungierenden Polypeptiden. Im Handel sind verschiedene Beads für zahlreiche Anwendungen erhältlich, überwiegend auf Kohlenhydrat (z. B. Agarose)-oder Kunststoffbasis. Sie enthalten aktive oder aktivierbare chemische Gruppen wie Carboxylgruppe, die für die Immobilisierung von Reagenzien genutzt werden können, z. B. von Antikörpern oder Antigenen. Bevorzugt handelt es sich um Beads mit einem durchschnittlichen Durchmesser von 0,2 mm bis 5 mm, 0,5 mm bis 1 mm, 0,75 mm bis 100 mm oder 1 mm bis 10 mm. Die Beads können mit einem Antigen beschichtet sein, das an einen diagnostisch relevanten Antikörper bindet, oder mit Affinitätsliganden, beispielsweise Biotin oder Glutathion. Bevorzugt umfasst die Flüssigkeit die Beads in Form einer wässrigen Suspension mit einem Beadgehalt von 10 bis 90 %, bevorzugter 20 bis 80, bevorzugter 30 bis 70, noch bevorzugter 40 bis 60 % (w/w).

In einer besonders bevorzugten Ausführungsform handelt es sich um paramagnetische Beads, die mit Hilfe eines Magneten leicht an einer Oberfläche konzentriert werden können. Zu diesem Zweck enthalten handelsübliche paramagnetische Beads meistens ein paramagnetisches Mineral, beispielsweise Eisenoxid.

Die Pufferflüssigkeit kann auch als Spülflüssigkeit bezeichnet werden. Derartige Pufferflüssigkeiten bzw. Spülflüssigkeiten sind dem Fachmann bekannt, insbesondere aus der EP 3 160 646 B1.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden. Obwohl manche Aspekte im Zusammenhang mit einer Vorrichtung insbesondere in Form eines Laborautomaten beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung bzw. eines Laborautomaten dar.

Je nach bestimmten Implementierungsanforderungen können Ausführungsbeispiele der Erfindung in Hardware oder in Software implementiert sein, insbesondere eine Steuereinheit und/oder eine Leseeinheit und/oder eine Lesestation. Eine programmierbare Hardwarekomponente kann insbesondere durch einen Microcontroller, einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) oder nur einem FPGA ohne Microcontroller gebildet sein.

## Patentansprüche

1. Vorrichtung zur Inkubation von mehreren Patientenproben unter Verwendung mehrerer Inkubationsgefäße,
aufweisend
- eine erste Einheit (E1) mit jeweiligen Aufnahmen (A) für jeweilige Inkubationsgefäße (IG),
- sowie eine zweite Einheit (E2) mit jeweiligen Aufnahmen (A) für jeweilige Inkubationsgefäße (IG),
wobei beide der jeweiligen Einheiten (E1, E2) entlang einer gemeinsamen Linearführungsachse (LFA) jeweils in ihrer jeweiligen Ortsposition bewegbar gelagert sind,
ferner aufweisend
- jeweilige Sensoren (S1, S2) zur Bereitstellung jeweiliger Sensorsignale (SIG1, SIG2), welche jeweils eine der jeweiliger Ortspositionen indizieren,
- ferner jeweilige Magnetantriebseinheiten (MA1, MA2) für jeweilige Änderungen der jeweiligen Ortspositionen,
- sowie ferner eine Steuereinheit (SE), welche ausgebildet ist, auf Basis der jeweiligen Sensorsignale (SIG1, SIG2) die jeweiligen Magnetantriebseinheiten (MA1, MA2) zur Herbeiführung der jeweiligen Änderungen der jeweiligen Ortspositionen anzusteuern.

2. Vorrichtung nach Anspruch 1,
wobei die beiden Einheiten (E1, E2) jeweils in der Weise entlang der gemeinsamen Linearführungsachse (LFA) gelagert und geführt sind, dass sie nur einen gemeinsamen translatorischen Freiheitsgrad besitzen.

3. Vorrichtung nach Anspruch 1,
wobei eine jeweilige Einheit (E1, E2) über eine jeweilige Federeinheit (FE1, FE2) mit einer gemeinsamen Grundplatte (GP) mechanisch gekoppelt ist.

4. Vorrichtung nach Anspruch 3,
wobei eine jeweilige Einheit (E1, E2) so über eine jeweilige Federeinheit (FE1, FE2) mit der gemeinsamen Grundplatte (GP) mechanisch gekoppelt ist, dass die jeweilige Einheit (E1, E2) gemeinsam mit der jeweiligen Federeinheit (FE1, FE2) ein jeweiliges Feder-Masse-Schwingsystem ausbildet.

5. Vorrichtung nach Anspruch 1,
wobei die Steuereinheit (SE) ausgebildet ist, die Magnetantriebseinheiten (MA1, MA2) so anzusteuern, dass die Einheiten (E1, E2) jeweils in Schwingung mit einer jeweils im Wesentlichen gleichen Schwingamplitude in Richtung der Linearführungsachse (LFA) gehalten werden.

6. Vorrichtung nach Anspruch 1,
wobei die Steuereinheit (SE) ausgebildet ist, die Magnetantriebseinheiten (MA1, MA2) mittels einer Regelung auf Basis der Sensorsignale (SIG1, SIG2) so anzusteuern, dass die sich ergebenden jeweiligen Schwingfrequenzen der jeweiligen Einheiten im Wesentlichen gleich sind und dass die jeweiligen Schwingfrequenzen einen Phasenversatz von im Wesentlichen 180 Grad zueinander aufweisen.

7. Vorrichtung nach Anspruch 1,
wobei die Steuereinheit (SE) ausgebildet ist, für eine jeweilige Einheit (E1, E2) auf Basis eines jeweiligen Sensorsignals (SIG1, SIG2) eine jeweilige aktuelle Amplitude und eine jeweilige Schwingfrequenz zu ermitteln,
und wobei die Steuereinheit (SE) ausgebildet ist, beide Einheiten (E1, E2) auf eine gemeinsame Schwingamplitude und eine gemeinsame, gleiche Ziel-Schwingfrequenz zu regeln.

8. Vorrichtung nach Anspruch 3 oder 4,
wobei beide Einheiten (E1, E2) mit den jeweiligen Federeinheiten (FE1, FE2) jeweils ein Feder-Masse-Schwingsystem mit einer jeweils gleichen Resonanzfrequenz (RF1) im unbeladenen Zustand aufweisen
und wobei die Ziel-Schwingfrequenz unterhalb dieser Resonanzfrequenz (RF1) liegt.

9. Vorrichtung nach Anspruch 1,
wobei die Steuereinheit (SE) ausgebildet ist, eine PI-Regelung bezogen auf eine gemeinsame, gleiche Ziel-Schwingfrequenz vorzunehmen.

10. Vorrichtung nach Anspruch 1,
wobei beide Einheiten (E1, E2) eine jeweilige Heizeinheit (H1, H2) aufweisen.

11. Laborautomat (LA) mit einer Vorrichtung (V) nach Anspruch 1,
ferner aufweisend
- ein Magazin (MAG) mit einer Mehrzahl von Inkubationsgefäßen (IG),
- ein Behältnis (B1) mit einer Patientenprobenflüssigkeit (PPF), ein Behältnis (B2) mit einer Pufferflüssigkeit (PUF), ein Behältnis (B3) mit einer Bead-Flüssigkeit (BF) aufweisend mit Antigenen oder mit Antikörpern beschichteten Beads sowie ein Behältnis (B4) mit einer Label-Flüssigkeit (AF) aufweisend mit einem Chemilumineszenzlabel markierte Antikörper oder Antigene,
- eine Pipettiereinheit (PE), welche ausgebildet ist, um wenigstens einen Teil der Patientenprobenflüssigkeit, wenigstens einen Teil der Pufferflüssigkeit, wenigstens einen Teil der Bead-Flüssigkeit und wenigstens einen Teil der Label-Flüssigkeit in die Inkubationsgefäße zu dispensieren und aus den Inkubationsgefäßen zu aspirieren,
- sowie ferner eine Greifeinheit (G), welche ausgebildet ist, Inkubationsgefäße
• aus dem Magazin (MAG) zur Pipettiereinheit (PE) zu transportieren
• sowie von der Pipettiereinheit (PE) in jeweilige der Aufnahmen (A) zu transportieren, aus den jeweiligen Aufnahmen (A) zu entnehmen und wieder hin zu der Pipettiereinheit (PE) zu transportieren.

12. Laborautomat nach Anspruch 11,
ferner aufweisend ein Behältnis (B5) mit einer Flüssigkeit aufweisend ein Enzym (E) zur Umsetzung einer Chemilumineszenzreaktion, insbesondere ferner aufweisend eine Dispensiereinheit zum Einbringen der Flüssigkeit aufweisend das Enzym in ein Inkubationsgefäß,
sowie ferner aufweisend eine Leseeinheit (LE) zur Erfassung eines optischen Signals (OS) einer Chemilumineszenzreaktion.

13. Laborautomat nach Anspruch 11,
wobei die Magnetantriebe (MA1, MA2) so angeordnet sind, dass in der Bead-Flüssigkeit (BF) enthaltene Beads nicht wesentlich durch Magnetfelder der Magnetantriebe (MA1, MA2) magnetisch beeinflusst werden.
